# EUROPÄISCHE PATENTSCHRIFT

(11) **EP 3 648 710 B1**
(45) Veröffentlichungstag und Bekanntmachung des Hinweises auf die Patenterteilung: **15.09.2021**
(21) Anmeldenummer: 18733228.3
(22) Anmeldetag: 19.06.2018
(51) Int. Cl.: A61F 2/66

(54) **PROTHESE UND PROTHESENFUSSADAPTER**
PROSTHESIS AND PROSTHETIC FOOT ADAPTER
PROTHÈSE ET ADAPTATEUR DE PIED PROTHÉTIQUE

(30) Priorität: 04.07.2017 DE 102017114892
(43) Veröffentlichungstag der Anmeldung: 13.05.2020
(73) Patentinhaber: Ottobock SE & Co. KGaA, 37115 Duderstadt (DE)
(72) Erfinder: ALLERMANN, Ralf, 37083 Göttingen (DE); VOSSLER, Sandra, 37085 Göttingen (DE); KUBITZ, Sven, 37085 Göttingen (DE); KALTENBORN, Sven, 37115 Duderstadt (DE); SCHWARTZ, Miclas, 37083 Göttingen (DE)
(74) Vertreter: Gramm, Lins & Partner Patent- und Rechtsanwälte PartGmbB
(86) Internationale Anmeldenummer: PCT/EP2018/066271
(87) Internationale Veröffentlichungsnummer: WO 2019/007678

(56) Entgegenhaltungen:
- DE-A1-102014 116 240
- DE-T2- 60 005 503
- GB-A- 2 177 925
- US-A1- 2016 331 559

## Beschreibung

Beschrieben wird hiermit eine Prothese mit einem Prothesenfuß mit einem Vorfußbereich und einem Fersenbereich, einem Prothesenunterschenkel und einem Prothesenfußadapter zum Anordnen des Prothesenfußes an dem Prothesenunterschenkel, wobei der Prothesenfußadapter ein Oberteil zum bezüglich einer Sagittalebene drehfesten Befestigen an dem Prothesenunterschenkel, ein Unterteil zum bezüglich der Sagittalebene drehfesten Befestigen an dem Prothesenfuß und ein Widerstandselement aufweist, wobei das Oberteil an dem Unterteil gegen einen von dem Widerstandselement aufgebrachten Widerstand um eine Schwenkachse verschwenkbar ist, die sich vom Fersenbereich zu dem Vorfußbereich erstreckt.

Derartige Prothesen sind aus dem Stand der Technik seit langem bekannt und werden beispielsweise in der DE 10 2014 116 240 A1 und der US 2016/0331559 A1 offenbart. Ziel derartiger Prothesen ist es, Personen, denen ein Bein oberhalb oder unterhalb des Knies amputiert werden musste, die Möglichkeit zu geben, mit der Prothese zu gehen und ein möglichst natürliches Gangbild bei größtmöglicher Stabilität und Sicherheit für den jeweiligen Träger der Prothese zu ermöglichen. Aus dem Stand der Technik sind dabei unterschiedlichste Ansätze bekannt, eine derartige Prothese zu konstruieren. Die US 6,764,521 B2 beispielsweise zeigt eine entsprechende Prothese. Der Prothesenfuß ist über einen Prothesenfußadapter an einem Prothesenunterschenkel angeordnet. Dabei ist der Prothesenfußadapter so ausgebildet, dass eine multiaxiale Verschwenkbarkeit des Prothesenfußes relativ zum Prothesenunterschenkel gewährleistet ist. Damit sollen die natürlichen Bewegungsmöglichkeiten eines gesunden Fußes nachgebildet werden. Eine Schwenkachse, die sich von medial nach lateral erstreckt, bildet dabei das Knöchelgelenk oder Sprunggelenk nach. Sie ermöglicht eine Bewegung des Fußes in Dorsalflexions- und Plantarflexionsrichtung. Zusätzlich ist eine zweite Rotations- oder Schwenkachse vorgesehen, die sich senkrecht zu dieser ersten Achse erstreckt und eine Verschwenkung des Fußes in medialer und lateraler Richtung ermöglicht. Damit soll gewährleistet werden, dass beispielsweise beim Auftreten auf geneigten Flächen, etwa beim Stehen an einem Hang, eine ausreichende Stabilität und ein vollflächiger Kontakt zum Boden ermöglicht wird.

Einen ähnlichen Ansatz verfolgt die DE 10 2004 037 877 A1. Auch hier wird eine Prothesenvorrichtung beschrieben, bei der ein Prothesenfuß an einem Prothesenunterschenkel angeordnet ist, wobei zwischen den beiden Bauteilen ein Kreuzgelenk angeordnet wird. Dieses verfügt über zwei senkrecht aufeinander stehende Drehachsen, die den bereits beschriebenen Achsen entsprechen und eine Bewegung des Fußes um zwei orthogonal aufeinander stehenden Schwenkachsen ermöglichen.

Nachteilig ist jedoch, dass der benötigte Bauraum für derartige Prothesen und insbesondere für Prothesenfußadapter für derartige Prothesen relativ groß ist, so dass insbesondere in einer Fußkosmetik ein derartiger Prothesenfußadapter gegebenenfalls nicht vollständig unterbringbar ist. Eine Prothesenkosmetik ist dabei beispielsweise eine Kunststoffhülle, die um den eigentlichen Prothesenfuß herum angeordnet wird, um ihm ein möglichst natürliches Aussehen zu verleihen.

Daher wurde in der US 7,044,984 B2 ein Prothesenfuß vorgestellt, der nicht über einen separaten Prothesenfußadapter verfügt. Stattdessen verfügt der Prothesenfuß über eine Aufnahme, in die ein Teil des Prothesenunterschenkels eingesetzt werden kann, so dass die beiden Bauteile um nur eine Achse schwenkbar miteinander verbunden sind. Diese Achse ermöglicht eine Verschwenkung des Prothesenfußes relativ zum Prothesenunterschenkel in Medial- und Lateralrichtung und erstreckt sich folglich vom Fersenbereich des Prothesenfußes zum Vorfußbereich. Um eine Bewegung des Knöchelgelenkes nachbilden zu können, besteht der Prothesenunterschenkel aus einem federnden Material, beispielsweise einem Kohlefaserverbundwerkstoff. Es ist folglich kein separates Gelenk vorhanden, sondern die Beweglichkeit wird durch die Elastizität und Federwirkung des Prothesenunterschenkels hervorgerufen.

Nachteilig bei allen beschriebenen Ausführungsformen ist, dass in bestimmten Bewegungssituationen ein Gefühl der Unsicherheit und Instabilität beim Träger der jeweiligen Prothese auftreten kann. Der Erfindung liegt daher die Aufgabe zugrunde, diese Nachteile zu beheben oder zumindest zu mildern.

Die Erfindung löst die gestellte Aufgabe durch einen Prothesenfußadapter, der sich dadurch auszeichnet, dass Rückstellelement wenigstens einen Torsionsstab aufweist und eine Prothese beinhaltend einen solchen Prothesenfußadapter, wie in den Ansprüchen beschrieben.

Der Erfindung liegt die überraschende Erkenntnis zugrunde, dass es für ein in vielen Fällen ausreichend natürliches Gangbild und eine erhöhte Stabilität und Sicherheit für den Prothesenträger ausreichend ist, wenn das Oberteil an dem Unterteil nur um eine Schwenkachse verschwenkbar angeordnet ist und ansonsten eine Verdrehung oder Bewegung des Prothesenunterschenkels relativ zum Prothesenfuß nicht vorgesehen ist. Auch der Prothesenunterschenkel muss nicht elastisch oder federnd ausgebildet sein. Eine Verschwenkung oder Bewegung des Prothesenfußes relativ zum Prothesenunterschenkel in Form einer Dorsalflexion oder Plantarflexion ist bei einer Prothese gemäß der vorliegenden Erfindung nicht notwendig und vorzugsweise nicht möglich. Dies bedeutet insbesondere, dass sich der Prothesenunterschenkel und die Lage der Schwenkachse relativ zu einer Längsachse des Prothesenunterschenkels, bei den Belastungen, die beim Gehen oder Stehen mit der Prothese durch den Prothesenträger auf die Prothese aufgebracht werden, nicht oder nur unwesentlich verformt oder verändert. Besonders vorteilhafterweise bleibt ein Winkel zwischen der Schwenkachse und der Längserstreckung des Prothesenunterschenkels in allen in Betrieb und bei der Benutzung der Prothese auftretenden Belastungssituationen nahezu konstant oder vollständig konstant.

Die Sagittalebene ist dabei eine Ebene, die durch eine Längsrichtung des Prothesenunterschenkels und einer Längsrichtung eines Prothesenfußes, der an dem Prothesenfußadapter anzuordnen ist, gebildet wird. Sie entspricht der aus der Orthopädie bekannten Sagittalebene am menschlichen Körper, in der die genannten Längsrichtungen liegen. Bei einem gesunden menschlichen Knöchel erlaubt das Knöchelgelenk eine derartige Drehung oder Verschwenkung innerhalb der Sagittalebene, die erfindungsgemäß nicht möglich ist. Eine Torsion oder eine Drehung um die Längsrichtung des Prothesenunterschenkels und/oder eine Verschiebung beispielsweise als Stoßdämpfung entlang der Längsrichtung des Prothesenunterschenkels sind vorzugsweise bei Prothesen der hier beschriebenen Art möglich.

Vorzugsweise ist das Widerstandselement ein Rückstellelement, das eine Rückstellkraft ausübt, ein Dämpfer, ein Federelement, beispielsweise eine Schraubenfeder oder ein Blattfeder, oder ein Reibelement, bei dem durch Reibung der Widerstand gegen die Verschwenkung aufgebracht wird. Insbesondere ein Rückstellelement oder ein Federelement hat sich als vorteilhaft herausgestellt, da beim Verschwenken durch diese Elemente eine Kraft aufgebracht wird, die das Oberteil und das Unterteil wieder in die ursprüngliche Position zueinander bringt oder zumindest eine derartige Verschwenkung in die der ursprünglichen Verschwenkung entgegengesetzte Richtung vereinfacht und unterstützt.

In einer bevorzugten Ausgestaltung verfügt der Prothesenunterschenkel über eine Längsachse und die Schwenkachse und die Längsachse schließen einen von 90° verschiedenen Winkel ein. Als besonders vorteilhaft hat sich herausgestellt, wenn die Schwenkachse in Richtung auf den Vorfußbereich geneigt ist. Dies bedeutet, dass ein Abstand der Schwenkachse relativ zu einem Boden, auf dem der Prothesenfuß aufliegt, im Vorfußbereich kleiner ist als im Fersenbereich.

Dadurch werden mehrere Vorteile erreicht. Da die Schwenkachse, um die das Oberteil relativ zum Unterteil und damit auch der an dem Oberteil befestigte Prothesenunterschenkel relativ zum am Unterteil befestigten Prothesenfuß verschwenkbar ist, nicht in der Auflagefläche des Prothesenfußes am Boden verläuft, hat ein Verschwenken des Prothesenfußes relativ zum Prothesenunterschenkel um diese Schwenkachse nicht nur eine Veränderung der Fußneigung, sondern auch eine Veränderung der Position der Auftrittsfläche des Prothesenfußes zur Folge. Beim Verschwenken um die Schwenkachse bewegt sich die Auftrittsfläche entlang einer Kreisbahn um die Schwenkachse herum. Je größer der Abstand zwischen der Schwenkachse und der Auftrittsfläche des Prothesenfußes ist, desto größer ist auch der Radius dieser Kreisbahn, so dass eine Verschwenkung um einen bestimmten Winkel einen deutlich größeren Einfluss auf die Verschiebung der Auftrittsfläche aufweist. Daher ist es von Vorteil, die Schwenkachse möglichst nah an die Auflagefläche des Fußes zu legen, um dem Prothesenträger nicht das Gefühl eines unsicheren Standes oder einer mangelnden Stabilität zu geben. Dies wird vorliegend vorzugsweise dadurch erreicht, dass die Schwenkachse in Richtung auf den Vorfußbereich geneigt ist, so dass in diesem Bereich der Abstand zwischen der Schwenkachse und der Auftrittsfläche besonders gering ist und daher die Stabilität in diesem Bereich als besonders hoch empfunden wird. Gleichzeitig ist durch die geneigte Anordnung der Schwenkachse im Fersenbereich des Prothesenfußes ausreichend Bauraum vorhanden, um, sofern dies gewünscht ist, Fersenfunktionen zu integrieren. Dies kann beispielsweise durch Federelemente, beispielsweise Blattfedern, oder Fersenpolster, beispielsweise einem Schaum oder Elastomer, geschehen, wie dies aus dem Stand der Technik bekannt ist, um beispielsweise die beim Fersenauftritt auftretenden Kräfte abdämpfen zu können.

Durch die geneigte Schwenkachse wird zudem der Bauraum, der innerhalb einer Fußkosmetik zur Verfügung steht, besser ausgenutzt, da auch dieser in Richtung auf den Vorfuß geneigt ist.

Ein weiterer Vorteil ist eine auftretende Kombinationsbewegung zwischen dem Prothesenunterschenkel und dem Prothesenfuß, die durch die geneigte Schwenkachse entsteht. Tritt der Prothesenträger beispielsweise mit dem Prothesenfuß auf eine geneigte Oberfläche, so dass sich im aufgetretenen und stabilen Zustand die Außenkante des Fußes abgesenkt hat, rotiert durch die geneigte Schwenkachse der Prothesenunterschenkel nach innen. Bei der entgegengesetzten Bewegung, bei der sich die Außenkante des Prothesenfußes gegenüber der Innenkante anhebt, wird der Prothesenunterschenkel nach außen rotiert. Die Stärke dieser gleichzeitigen Rotation hängt vom Winkel zwischen der Schwenkachse und der Längsachse des Prothesenunterschenkels ab. Dieser Winkel beträgt vorteilhafterweise zwischen 95° und 120°, besonders bevorzugt zwischen 100° und 110°, besonders bevorzugt 105°.

Diese Kombinationsbewegung hat mehrere Effekte, die jeweils vorteilhaft sind. Beim Gehen auf der Ebene dreht sich am Beginn der Standphase eines Gangzykluses der Prothesenunterschenkel relativ zum Fuß einwärts und im Laufe des Abrollens nimmt er die Neutralstellung wieder ein. Dreht sich der Prothesenunterschenkel zum Beginn der Standphase einwärts, bewirkt die geneigte Schwenkachse eine Inversion des Prothesenfußes, so dass sich die Außenkante des Prothesenfußes absenkt. Der Krafteinleitungspunkt des Fußes wird daher in Richtung auf die Außenkante des Fußes verschoben, was den natürlichen Verlauf bei einem gesunden Fuß entspricht. Beim Abrollen des Fußes wird der Prothesenunterschenkel wieder in die Neutralstellung zurückgedreht, so dass auch die Inversion kompensiert wird und der Krafteinleitungspunkt wieder in den mittleren Bereich des Fußes in Medial-/ Lateralrichtung wandert, wenn er in den Bereich des Vorfußes durch das Überrollen des Fußes verschoben wird.

Der zweite Effekt der Kombinationsbewegung betrifft das Gehen oder Stehen an einem Hang. Durch den Hang wird der eine Fuß in die Inversion gedrückt, bei der die Außenkante des Fußes abgesenkt ist. Der jeweils andere Fuß wird in die Evasion gedrückt, bei der die Außenkante relativ zur Innenkante des Prothesenfußes angehoben ist. Dadurch kommt es zu einer entsprechenden Rotation des Prothesenunterschenkels, die am Hang immer bergauf erfolgt, wodurch eine zusätzliche Sicherheit erreicht wird.

Durch diese besonders bevorzugte Ausgestaltung einer in Richtung auf den Vorfußbereich geneigten Schwenkachse wird folglich die Sicherheit am Hang erhöht und zudem das Gangbild dem eines natürlichen Fußes angeglichen.

Erfindungsgemäß weist das Rückstellelement wenigstens einen Torsionsstab auf. Dieser ist beispielsweise ein Stab aus einem Material, dass beim Verdrillen, also unter einer Torsion, eine Rückstellkraft erzeugt. Der Stab kann beispielsweise aus Titan oder einem anderen Metall bestehen oder dieses zumindest beinhalten. Wird eine ausreichende Steifigkeit und Festigkeit erreicht, kann auch ein elastisches gegebenenfalls nichtmetallisches Material verwendet werden. Er ist so angeordnet, dass ein Verschwenken des Oberteils relativ zum Unterteil zumindest auch zu einer Verdrehung des Torsionsstabes führt, wodurch eine Rückstellkraft erzeugt wird. Selbstverständlich sind auch andere Rückstellelemente, beispielsweise Druckfedern, Zugfedern, Elastomerbauteile oder sonstige Dämpfer möglich. Auch beispielsweise gleichpolig angeordnete Magnete, die einander folglich abstoßen, können als Rückstellelemente verwendet werden. Selbstverständlich können beispielsweise auch einstellbare Elektromagnete oder Kombinationen mehrerer der genannten Lösungen als Rückstellelement verwendet werden. Der Vorteil von Druck- und Zugfedern und selbstverständlich auch kombinierten Zug-Druck-Federn sowie Elastomerbauteilen, Metallstäben wie beispielsweise auch den Torsionsstab, liegt darin, dass die Rückstellkraft mit steigendem Verschwenkungswinkel ansteigt. Je stärker folglich das Oberteil relativ zum Unterteil verschwenkt wird, desto größer wird die Rückstellkraft. Dies ist auch mit magnetischen Rückstellelementen zu erreichen, die beispielsweise aus sich abstoßend angeordneten Magneten bestehen. Dies können kaskadenförmig angeordnet werden, so dass bei stärkerer Verschwenkung des Oberteils relativ zum Unterteil mehr Magnete in den jeweiligen Wirkbereich eines gegenüberliegenden gleichpolig angeordneten Magneten kommen. Dadurch wird die Rückstellkraft erhöht. Die Rückstellkraft wird jedoch bereits dadurch erhöht, dass sich beispielsweise ein Abstand zwischen dem gleichpolig angeordneten Magneten bei steigender Verschwenkung des Oberteils relativ zum Unterteil verringert.

Ein Torsionsstab benötigt sehr wenig Bauraum und ist daher insbesondere für Prothesen, die einen kleinen Prothesenfuß aufweisen, beispielsweise für Kinderprothesen, von Vorteil. Selbstverständlich kann er jedoch auch bei anderen Prothesen vorteilhaft eingesetzt werden. Dabei ist er vorteilhafterweise so angeordnet, dass die Schwenkachse in seinem Inneren verläuft. Dies bedeutet nicht, dass die Schwenkachse ein separates Bauteil ist, das innerhalb des Torsionsstabes angeordnet ist. Die Schwenkachse ist in diesem Fall lediglich eine gedankliche Konstruktion. Beim Verschwenken des Oberteils relativ zum Unterteil kommt es dabei lediglich zu einer Verdrillung des Torsionsstabes. Alternativ oder zusätzlich dazu kann auch wenigstens ein Torsionsstab außerhalb der Schwenkachse angeordnet werden, so dass es zusätzlich zu der Verdrillung oder Torsion auch zu einer Verbiegung kommt, wodurch zusätzliche Rückstellkräfte hervorgerufen werden. Vorzugsweise ist der Torsionsstab in einer Hohlwelle angeordnet, die vorzugsweise mit dem Oberteil oder dem Unterteil drehfest verbunden ist. Vorteilhafterweise ist der Torsionsstab mit der Hohlwelle zumindest auch formschlüssig verbunden. Besonders bevorzugt ist er ausschließlich formschlüssig verbunden. Dies kann beispielsweise durch einen Mehrkant, insbesondere einen Dreikant und eine Schraube, insbesondere eine Madenschraube geschehen. Die Anordnung in einer Hohlwelle schützt die Mechanik vor Umwelteinflüssen und reduziert eine mögliche Klemmgefahr.

Vorzugsweise verfügt der wenigstens eine Torsionsstab über einen kreisförmigen Querschnitt. Dadurch wird eine gleichmäßige Verteilung der Schubspannung auf der Mantelfläche des Torsionsstabes erreicht.

In einer bevorzugten Ausgestaltung wird eine Verschwenkung des Oberteils relativ zum Unterteil in zumindest eine Richtung, bevorzugt in beide Schwenkrichtungen, durch wenigstens einen Anschlag, bevorzugt zwei Anschläge, begrenzt. Diese können beispielsweise erreicht werden, wenn das Oberteil so weit relativ zu dem Unterteil verschwenkt wird, dass es an dem Unterteil anschlägt. Selbstverständlich können auch separate Anschläge vorhanden sein, die vorzugsweise einstellbar sind, so dass ein maximaler Schwenkwinkel und/oder die Größe des Schwenkbereichs einstellbar sind. Zusätzlich verfügt der Prothesenfußadapter vorzugsweise über wenigstens ein Anschlagselement, bevorzugt wenigstens zwei Anschlagselemente, durch die ein Anschlagen an einem oder beiden Anschlägen gedämpft wird. Diese können als harte Anschlagselemente ausgebildet sein oder einen gewissen Dämpfungsgrad aufweisen, wenn sie beispielsweise in Form eines Federelementes oder eines elastischen Elementes ausgebildet sind. Die Anschlagselemente sowie das Rückstellelement können vollständig in einem Gehäuse oder vom Oberteil oder vom Unterteil umgeben sein, so dass beispielsweise eine Verschmutzung nahezu ausgeschlossen ist.

In einer bevorzugten Ausgestaltung sind das Widerstandselement und insbesondere der von ihm aufgebrachte Widerstand und/oder das wenigstens eine Anschlagselement austauschbar und/oder einstellbar.

Vorzugsweise verfügt die Prothese über eine Sperreinrichtung, die in eine Sperrstellung bringbar ist, in der ein Verschwenken des Oberteils relativ zu dem Unterteil nicht möglich ist. Die Sperreinrichtung kann beispielsweise einen Keil aufweisen, der verschiebbar oder drehbar ist und in wenigstens eine Position oder Orientierung relativ zum Rest der Prothese das Verschwenken verhindert. Selbstverständlich kann die Sperreinrichtung auch hydraulisch, pneumatisch oder elektronisch ausgebildet sein.

Die Erfindung löst die gestellte Aufgabe zudem durch einen Prothesenfußadapter für eine hier beschriebene Prothese.

Mit Hilfe der beiliegenden Zeichnung wird nachfolgend ein Ausführungsbeispiel der vorliegenden Erfindung näher erläutert. Es zeigt:
- Figur 1: - die schematische Schnittdarstellung durch eine Prothese gemäß einem ersten Ausführungsbeispiel der vorliegenden Erfindung,
- Figur 2: - eine schematische dreidimensionale Ansicht der Prothese,
- Figur 3: - die schematische Draufsicht auf einen Prothesenfuß mit Prothesenfußadapter gemäß einem Ausführungsbeispiel der vorliegenden Erfindung,
- Figur 4: - die schematische Schnittdarstellung durch einen Prothesenfußadapter,
- Figur 5: - eine Schnittdarstellung durch den Prothesenfußadapter entlang einer anderen Ebene,
- Figur 6: - die schematische Schnittdarstellung durch einen künstlichen Fuß,
- Figur 7: - die schematische Schnittdarstellung durch eine spezielle Ausgestaltung eines Rückstellelementes und
- Figur 8: - die schematische Darstellung einer Rückstellkraft.

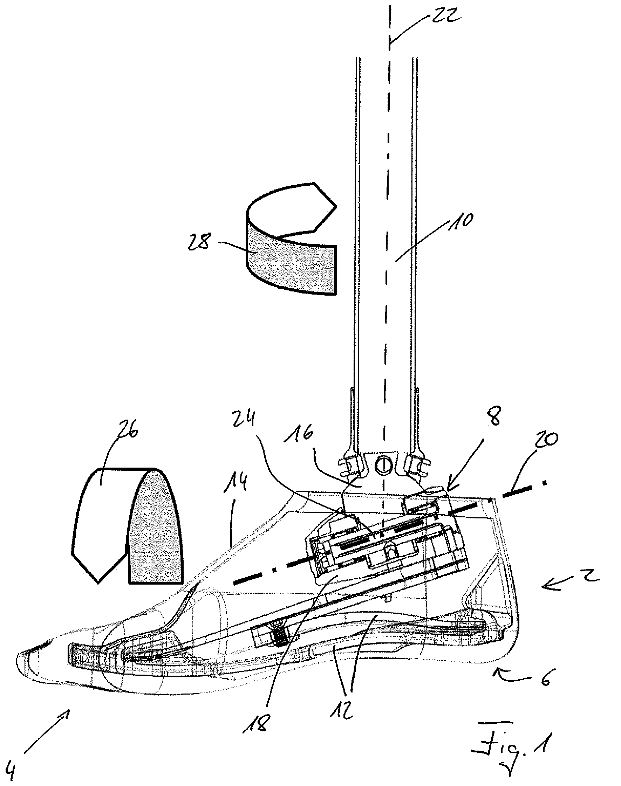

Figur 1 zeigt eine schematische Schnittdarstellung durch eine Prothese gemäß einem ersten Ausführungsbeispiel der vorliegenden Erfindung. Sie verfügt über einen Prothesenfuß 2, der über einen Vorfußbereich 4 und einen Fersenbereich 6 verfügt. Der Prothesenfuß 2 ist über einen Prothesenfußadapter 8 mit einem Prothesenunterschenkel 10 verbunden.

Der Prothesenfuß 2 verfügt über mehrere Blattfedern 12, die die federnden Eigenschaften des Prothesenfußes 2 bestimmen. Sie sind von einer Fußkosmetik 14 umgeben. Durch sie, die dem Prothesenfuß 2 ein möglichst natürliches Aussehen verleihen soll, wird der für den Prothesenfußadapter 8 zur Verfügung stehende Bauraum begrenzt.

Der Prothesenfußadapter 8 verfügt über ein Oberteil 16, an dem der Prothesenunterschenkel 10 drehfest angeordnet ist. Zudem verfügt er über ein Unterteil 18, das drehfest mit dem Prothesenfuß 2 verbunden ist. Das Oberteil 16 ist relativ zum Unterteil 18 um eine Schwenkachse 20 herum schwenkbar, die in Figur 1 relativ zu einer Längsachse 22 des Prothesenunterschenkels 10 geneigt ist und keinen rechten Winkel einschließt.

Im Inneren des Prothesenfußadapters 8 befindet sich ein Rückstellelement 24, durch das eine Rückstellkraft aufgebracht wird, wenn das Oberteil 16 relativ zum Unterteil 18 verschwenkt wird.

Da die Schwenkachse 20 geneigt ist, führt eine Verschwenkung des Prothesenfußes 2 in Richtung des ersten Pfeils 26 im belasteten Zustand der Prothese dazu, dass der Prothesenunterschenkel 10 in Richtung des zweiten Pfeils 28 gedreht wird. Diese Kombinationsbewegung wurde bereits detaillierter beschrieben.

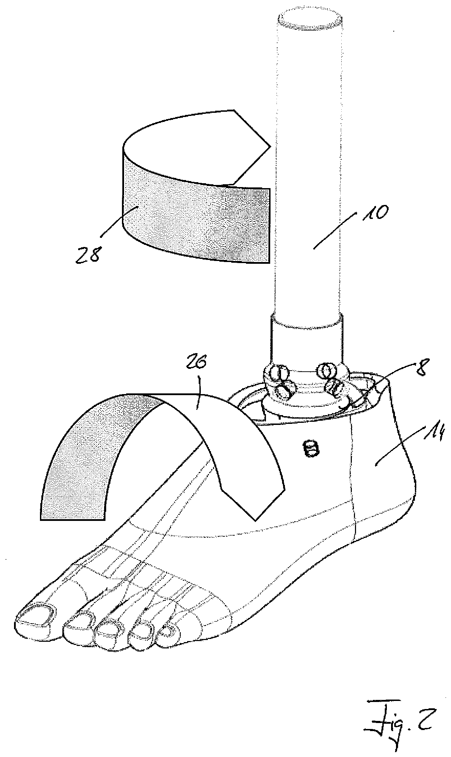

Figur 2 zeigt die Prothese in einer schematischen 3D-Ansicht. Man erkennt die Fußkosmetik 14 sowie den Prothesenunterschenkel 10, der am Prothesenfußadapter 8 angeordnet ist. Eine Rotation des Prothesenfußes 2 in Richtung des ersten Pfeils 26 führt dazu, dass der Prothesenunterschenkel 10 in Richtung des zweiten Pfeils 28 rotiert wird, wenn, wie im gezeigten Ausführungsbeispiel, die Schwenkachse 20 relativ zur Auflagefläche des Prothesenfußes geneigt ist und somit mit der Längsachse 22 des Prothesenunterschenkels 10 keinen rechten Winkel einschließt.

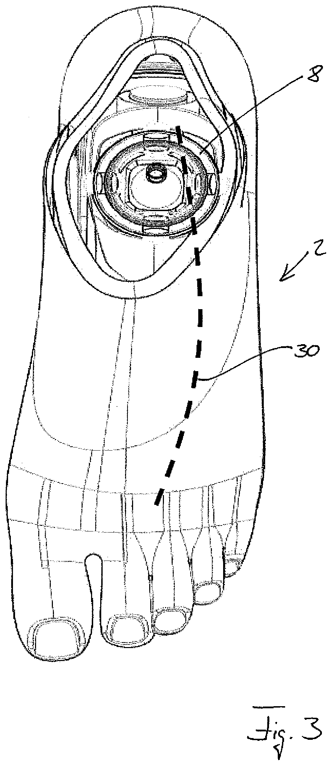

Figur 3 zeigt den Prothesenfuß 2 mit dem Prothesenfußadapter 8 in einer schematischen Draufsicht. Durch die gestrichelte Linie 30 wird der Verlauf des Krafteinleitungspunktes im Verlauf eines Schrittes schematisch dargestellt. Man erkennt, dass der Verlauf deutlich näher an dem natürlichen Verlauf ist, bei dem ebenfalls der Krafteinleitungspunkt in Richtung auf die laterale Seite des Fußes, also zur Außenkante hin, verschoben ist, als dies bei herkömmlichen Prothesenfüßen der Fall ist.

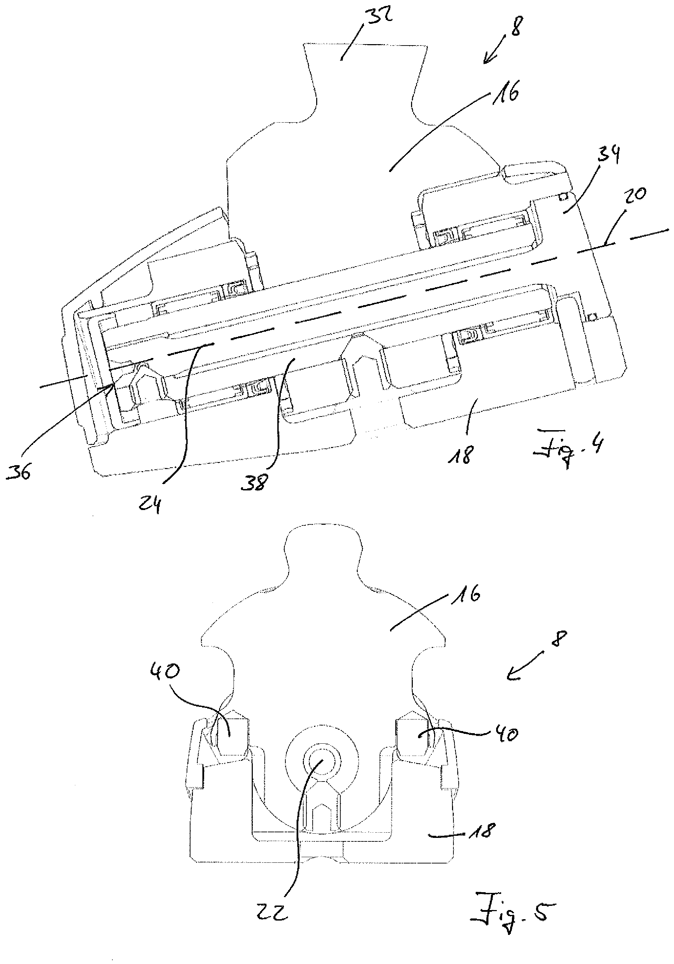

Figur 4 zeigt eine vergrößerte Schnittdarstellung durch den Prothesenfußadapter 8. An einem Befestigungselement 32 kann der Prothesenunterschenkel 10 angeordnet werden. Das Befestigungselement 32 ist Teil des Oberteils 16, das relativ zum Unterteil 18 um die Schwenkachse 20 schwenkbar gelagert ist. Im Inneren befindet sich ein Rückstellelement 24 in Form eines Torsionsstabes. Dieser ist an seinem fersenseitigen Ende 34 mit dem Unterteil 18 drehfest verbunden und an seinem vorfußseitigen Ende 36 drehfest am Oberteil 16 befestigt. Werden nun das Oberteil 16 und das Unterteil 18 relativ zueinander verschwenkt, führt dies zu einer Torsion des in einer Hohlwelle 38 gelagerten Rückstellelementes 24 und damit zu einer aufgebrachten Rückstellkraft.

Figur 5 zeigt eine schematische Schnittdarstellung in einer Ebene senkrecht zu der Schnittebene aus Figur 4. Man erkennt das Oberteil 16, das Unterteil 18 sowie die Längsachse 22, um die das Oberteil 16 relativ zum Unterteil 18 verschwenkbar ist. Zusätzlich verfügt der in Figur 5 gezeigte Prothesenfußadapter 8 über zwei Anschlagselemente 40, durch die ein Anschlagen des Oberteils 16 am Unterteil 18, das beim Erreichen eines maximalen Schwenkwinkel in beide Richtungen auftreten kann, gedämpft wird.

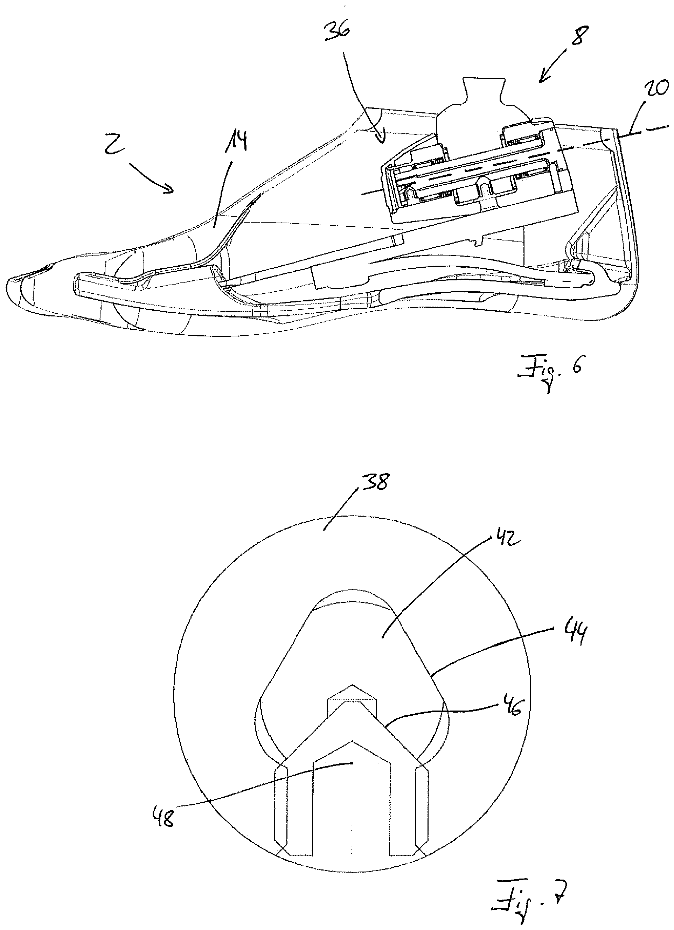

Figur 6 zeigt den in Figur 4 dargestellten Prothesenfußadapter 8 in einem Prothesenfuß 2. Durch die Neigung der Schwingachse 20 passt der Prothesenfußadapter 8 insbesondere in seinem vorfußseitigen Ende 36 besser in einen durch die Fußkosmetik 14 zur Verfügung gestellten Bauraum.

Figur 7 zeigt eine Schnittdarstellung senkrecht zur Erstreckungsrichtung des Rückstellelementes 24. Das Rückstellelement 24 ist als Torsionsstab 42 ausgebildet und in einer Hohlwelle 38 angeordnet. Diese verfügt über einen Innendreikant 44 dem sich die Außenform des Torsionsstabes 42 mit einem Außendreikant anpasst. Der Torsionsstab 42 verfügt zudem über eine konische Bohrung 46 in dem angezeigten Ausführungsbeispiel einer Madenschraube 48 oder ein Gewindestift angeordnet ist.

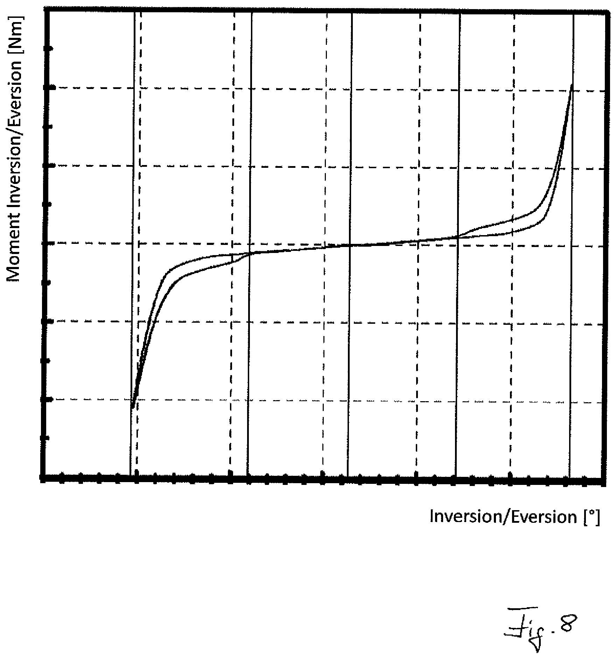

Figur 8 zeigt den Zusammenhang zwischen einer Inversion oder Eversion des Fußes, also einer Verschwenkung um die Schwenkachse 20, die auf der horizontalen Achse, also in dem Diagramm der x-Achse aufgetragen ist, und einem entsprechend hervorgerufenen Drehmoment auf der vertikalen Achse, also der y-Achse. Man erkennt, dass insbesondere durch das aktivieren und/oder deaktivieren zusätzlicher Torsionselement oder Rückstellelemente 24 ein nahezu beliebiger Zusammenhang hergestellt werden kann. Im gezeigten Ausführungsbeispiel wächst der Eversions- und Inversionswiderstand zunächst linear an. Nach etwa der Hälfte des Winkelbereichs setzt eine Progression an, so dass schließlich eine weitere Inversion oder weitere Eversion über den Grenzwinkel hinaus nicht oder nur schwer möglich ist.

### Bezugszeichenliste

- 2: Prothesenfuß
- 4: Vorfußbereich
- 6: Fersenbereich
- 8: Prothesenfußadapter
- 10: Prothesenunterschenkel

- 12: Blattfeder
- 14: Fußkosmetik
- 16: Oberteil
- 18: Unterteil
- 20: Schwenkachse

- 22: Längsachse
- 24: Rückstellelement
- 26: erster Pfeil
- 28: zweiter Pfeil
- 30: gestrichelte Linie

- 32: Befestigungselement
- 34: fersenseitiges Ende
- 36: vorfußseitiges Ende
- 38: Hohlwelle
- 40: Anschlagselement
- 42: Torsionsstab
- 44: Innendreikant
- 46: konische Bohrung
- 48: Madenschraube

## Patentansprüche

1. Prothesenfußadapter (8) zum Anordnen eines Prothesenfußes (2) mit einem Vorfußbereich (4) und einem Fersenbereich (6) an einem Prothesenunterschenkel (10), wobei der Prothesenfußadapter (8) - ein Oberteil (16) zum bezüglich einer Sagittalebene drehfesten Befestigen an dem Prothesenunterschenkel (10), - ein Unterteil (18) zum bezüglich der Sagittalebene drehfesten Befestigen an dem Prothesenfuß (2) und - ein Widerstandselement (24) aufweist, wobei das Oberteil (16) an dem Unterteil (18) gegen einen von dem Widerstandselement (24) aufgebrachten Widerstand um eine Schwenkachse (20) verschwenkbar ist, die sich, im angeordneten Zustand, vom Fersenbereich (6) zu dem Vorfußbereich (4) erstreckt, wobei das Widerstandselement (24) ein Rückstellelement ist, **dadurch gekennzeichnet, dass** das Rückstellelement (24) wenigstens einen Torsionsstab aufweist.

2. Prothesenfußadapter (8) nach Anspruch 1, **dadurch gekennzeichnet, dass** der wenigstens eine Torsionsstab in einer Hohlwelle (38) angeordnet ist, die vorzugsweise drehfest mit dem Oberteil (16) oder dem Unterteil (18) verbunden ist.

3. Prothesenfußadapter (8) nach Anspruch 2, **dadurch gekennzeichnet, dass** der Torsionsstab zumindest auch formschlüssig mit der Hohlwelle (38) verbunden ist.

4. Prothesenfußadapter nach einem der vorstehenden Ansprüche, **dadurch gekennzeichnet, dass** der wenigstens eine Torsionsstab einen kreisförmigen Querschnitt aufweist.

5. Prothesenfußadapter nach einem der vorstehenden Ansprüche, **dadurch gekennzeichnet, dass** eine Verschwenkung des Oberteiles (16) relativ zum Unterteil (18) in zumindest eine Richtung, bevorzugt in zwei Richtungen durch wenigstens einen Anschlag, bevorzugt zwei Anschläge, begrenzt wird und der Prothesenfußadapter (8) wenigstens ein Anschlagselement (40), bevorzugt wenigstens zwei Anschlagselemente (40), aufweist, durch die ein Anschlagen gedämpft wird.

6. Prothesenfußadapter nach einem der vorstehenden Ansprüche, **dadurch gekennzeichnet, dass** das Widerstandselement (24) und oder das wenigstens eine Anschlagselement (40) austauschbar und/ oder einstellbar sind.

7. Prothese mit
- einem Prothesenfußadapter nach einem der vorstehenden Ansprüche,
- einem Prothesenfuß (2) mit einem Vorfußbereich (4) und einem Fersenbereich (6) und
- einem Prothesenunterschenkel (10).

8. Prothese nach Anspruch 7, **dadurch gekennzeichnet, dass** der Prothesenunterschenkel (10) eine Längsachse (22) aufweist und die Schwenkachse (20) und die Längsachse (22) einen von 90° verschiedenen Winkel einschließen.

9. Prothese nach Anspruch 8, **dadurch gekennzeichnet, dass** die Schwenkachse (20) in Richtung auf den Vorfußbereich (4) geneigt ist.

10. Prothese nach Anspruch 7-9, **dadurch gekennzeichnet, dass** die Prothese eine Sperreinrichtung aufweist, die in eine Sperrstellung bringbar ist, in der ein Verschwenken des Oberteils (16) relativ zu dem Unterteil (18) verhindert wird.

## Claims

1. A prosthesis foot adapter (8) for arranging a prosthetic foot (2) with a forefoot area (4) and a heel area (6) on a prosthetic lower leg (10),
wherein the prosthetic foot adapter (8) comprises
- an upper part (16) for fixing it to the prosthetic lower leg (10) such that it is torque-proof in relation to a sagittal plane,
- a lower part (18) for fixing it to the prosthetic foot (2) such that it is torque-proof in relation to a sagittal plane, and
- a resistance element (24),
wherein the upper part (16) can be swivelled on the lower part (18) about a swivel axis (20) against a resistance exerted by the resistance element (24), said swivel axis extending from the heel area (6) to the forefoot area (4) when the applied state, wherein the resistance element (24) is a restoring element, **characterized in that** restoring element (24) comprises at least one torsion bar.

2. The prosthesis foot adapter (8) according to claim 1, **characterized by the fact that** the at least one torsion bar is arranged inside a hollow shaft (38), which is preferably connected to the upper part (16) or the lower part (18) such that it is torque-proof..

3. The prosthesis foot adapter (8) according to claim 2, **characterized by the fact that** the torsion bar is at least also positively connected to the hollow shaft (38).

4. The prosthesis foot adapter (8) according to one of the above claims, **characterized by the fact that** the at least one torsion bar has a circular cross-section.

5. The prosthesis foot adapter (8) according to one of the above claims, **characterized by the fact that** a swivelling of the upper part (16) relative to the lower part (18) in at least one direction, preferably in two directions, is restricted by at least one end stop, preferably by two end stops, and the prosthetic foot adapter (8) comprises at least one end stop element (40), preferably at least two end stop elements (40), by way of which a strike is damped.

6. The prosthesis foot adapter (8) according to one of the above claims, **characterized by the fact that** the resistance element (24) and/or the at least one end stop element (40) can be replaced and/or adjusted

7. A prosthesis comprising a prosthetic foot adapter according to one of the above claims, a prosthetic foot (2) with a forefoot area (4) and a heel area (6) and a prosthetic lower leg (10).

8. The prosthesis according to claim 7, **characterized by the fact that** the prosthetic lower leg (10) has a longitudinal axis (22), and the swivel axis (20) and the longitudinal axis (22) form an angle that is not 90°.

9. The prosthesis according to claim 8, **characterized by the fact that** the swivel axis (20) is tilted towards the forefoot area (4).

10. The prosthesis according to one of claims 7 to 9, **characterized by the fact that** the prosthesis features a locking device which can be brought into a locked position, in which a swivelling of the upper part (16) relative to the lower part (18) is prevented.

## Revendications

1. Adaptateur de pied prothétique (8) pour agencer un pied prothétique (2), pourvu d'une zone d'avant-pied (4) et d'une zone de talon (6), au niveau d'un bas de jambe prothétique (10), l'adaptateur de pied prothétique (8) comprenant une partie supérieure (16) destinée à être fixée au bas de jambe prothétique (10) solidairement en rotation par rapport à un plan sagittal, une partie inférieure (18) destinée à être fixée au pied prothétique (2) solidairement en rotation par rapport au plan sagittal, et un élément de résistance (24),
dans lequel, à l'encontre d'une résistance appliquée par l'élément de résistance (24), la partie supérieure (16) est mobile en pivotement au niveau de la partie inférieure (18) autour d'un axe de pivotement (20) qui, dans l'état agencé, s'étend de la zone de talon (6) à la zone d'avant-pied (4), l'élément de résistance (24) étant un élément de rappel,
**caractérisé en ce que** l'élément de rappel (24) comprend une barre de torsion.

2. Adaptateur de pied prothétique (8) selon la revendication 1,
**caractérisé en ce que** ladite au moins une barre de torsion est disposée dans un arbre creux (38) qui est relié de préférence solidairement en rotation à la partie supérieure (16) ou à la partie inférieure (18).

3. Adaptateur de pied prothétique (8) selon la revendication 2,
**caractérisé en ce que** la barre de torsion est reliée au moins également par coopération de forme à l'arbre creux (38).

4. Adaptateur de pied prothétique selon l'une des revendications précédentes,
**caractérisé en ce que** ladite au moins une barre de torsion présente une section transversale circulaire.

5. Adaptateur de pied prothétique selon l'une des revendications précédentes,
**caractérisé en ce qu'**un pivotement de la partie supérieure (16) par rapport à la partie inférieure (18) est limité dans au moins une direction, de préférence dans deux directions, par au moins une butée, de préférence par deux butées, et
l'adaptateur de pied prothétique (8) présente au moins un élément de butée (40), de préférence au moins deux éléments de butée (40), permettant d'amortir une venue en butée.

6. Adaptateur de pied prothétique selon l'une des revendications précédentes,
**caractérisé en ce que** l'élément de résistance (24) et/ou ledit au moins un élément de butée (40) sont interchangeables et/ou réglables.

7. Prothèse, comportant
- un adaptateur de pied prothétique selon l'une des revendications précédentes,
- un pied prothétique (2) pourvu d'une zone d'avant-pied (4) et d'une zone de talon (6), et
- un bas de jambe prothétique (10).

8. Prothèse selon la revendication 7,
**caractérisée en ce que** le bas de jambe prothétique (10) présente un axe longitudinal (22), et l'axe de pivotement (20) et l'axe longitudinal (22) forment un angle différent de 90°.

9. Prothèse selon la revendication 8,
**caractérisée en ce que** l'axe de pivotement (20) est incliné en direction de la zone d'avant-pied (4).

10. Prothèse selon l'une des revendications 7 à 9,
**caractérisée en ce que** la prothèse comprend un dispositif de blocage qui peut être amené dans une position de blocage dans laquelle un pivotement de la partie supérieure (16) par rapport à la partie inférieure (18) est empêché.
